# EUROPEAN PATENT APPLICATION

(11) **EP 4 616 849 A1**
(43) Date of publication of application: **17.09.2025**
(21) Application number: 23888104.9
(22) Date of filing: 10.11.2023
(51) Int. Cl.: A61K 9/10, A61K 31/58, A61K 47/36

(54) **TRIAMCINOLONE ACETONIDE COMPOSITION AND PREPARATION METHOD THEREFOR**

(30) Priority: 11.11.2022 CN 202211414846
(71) Applicant: Beijing Sightnovo Medical Technology Co., Ltd, Haidian District Beijing 100192 (CN)
(72) Inventor: XIA, Chaoran, Beijing 100192 (CN); ZHONG, Weixing, Beijing 100192 (CN); ZHANG, Miao, Beijing 100192 (CN)
(74) Representative: Gille Hrabal Partnerschaftsgesellschaft mbB Patentanwälte
(86) International application number: PCT/CN2023/130899
(87) International publication number: WO 2024/099419

(57) **Abstract**

A triamcinolone acetonide pharmaceutical composition and a preparation method therefor. The pharmaceutical composition contains the following ingredients: (i) a triamcinolone acetonide active ingredient, (ii) hyaluronic acid or a salt thereof. (iii) a buffering agent; and (iv) a tonicity adjustment agent. The pharmaceutical composition has a suitable viscosity, osmotic pressure and particle size range and is easily injected, especially administrated by means of a suprachoroidal space injection.

## Description

The present application claims priority to the prior application with the patent application No. 202211414846.0, entitled "TRIAMCINOLONE ACETONIDE COMPOSITION AND PREPARATION METHOD THEREFOR", and filed with the China National Intellectual Property Administration on November 11, 2022, which is incorporated herein by reference in its entirety.

### TECHNICAL FIELD

The present disclosure relates to the field of pharmaceutical formulations, and particularly relates to a triamcinolone acetonide composition and a preparation method therefor.

### BACKGROUND

The chemical name of triamcinolone acetonide is 9α-fluoro-11β,16α,17,21-tetrahydroxypregna-1,4-diene-3,20-dione cyclic 16,17-acetal with acetone, and its chemical structure is:

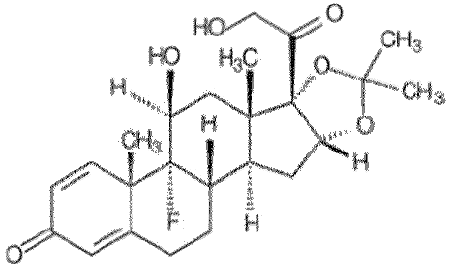

Triamcinolone acetonide is a long-acting adrenal glucocorticoid that is widely used in clinical practice due to its strong anti-inflammatory and anti-allergic effects.

XIPERE^{™}, submitted by BAUSCH AND LOMB INC, is the first product for suprachoroidal space (SCS) injection approved by the FDA in October 2021. Each milliliter of its sterile aqueous suspension contains 40 mg of triamcinolone acetonide, 0.55% (w/v) sodium chloride, 0.5% (w/v) sodium carboxymethylcellulose, and 0.02% (w/v) polysorbate-80. It also contains potassium chloride, calcium chloride (dihydrate), magnesium chloride (hexahydrate), sodium acetate (trihydrate), sodium citrate (dihydrate), and water for injection.

There is an urgent clinical need for an ophthalmic formulation with triamcinolone acetonide as an active pharmaceutical ingredient that features a simple manufacturing process and is suitable for topical administration, particularly for suprachoroidal space injection.

### SUMMARY

In order to solve the technical problems described above, the present disclosure provides in a first aspect a pharmaceutical composition comprising the following ingredients:
(i) a triamcinolone acetonide active ingredient;
(ii) hyaluronic acid or a salt thereof;
(iii) a buffering agent; and
(iv) a tonicity adjustment.

According to an embodiment of the present disclosure, the salt of hyaluronic acid may be selected from a hyaluronic acid metal salt, preferably from one or more of a hyaluronic acid alkali metal salt and a hyaluronic acid alkaline earth metal salt; more preferably, the salt of hyaluronic acid is selected from sodium hyaluronate.

According to an embodiment of the present disclosure, the (i) triamcinolone acetonide active ingredient comprises triamcinolone acetonide or triamcinolone acetonide acetate, or a pharmaceutically acceptable salt or solvate thereof. In certain embodiments, the (i) is selected from triamcinolone acetonide; in certain embodiments, the (i) is selected from triamcinolone acetonide acetate.

According to an embodiment of the present disclosure, the buffering agent is selected from one or more of an acetate, a citrate, a phosphate, and a borate; preferably, the buffering agent is selected from a phosphate buffering agent; more preferably, the buffering agent is selected from one or a combination of two of disodium hydrogen phosphate and sodium dihydrogen phosphate, wherein the sodium dihydrogen phosphate may be selected from sodium dihydrogen phosphate monohydrate, and the disodium hydrogen phosphate may be selected from disodium hydrogen phosphate dodecahydrate. According to an embodiment of the present disclosure, the tonicity adjustment is selected from one or a combination of two of sodium chloride and potassium chloride.

According to an embodiment of the present disclosure, the hyaluronic acid or the salt thereof has a molecular weight of 50,000 daltons to 2,000,000 daltons, e.g., 50,000 daltons, 60,000 daltons, 70,000 daltons, 80,000 daltons, 90,000 daltons, 100,000 daltons, 110,000 daltons, 120,000 daltons, 130,000 daltons, 140,000 daltons, 150,000 daltons, 160,000 daltons, 170,000 daltons, 180,000 daltons, 190,000 daltons, 200,000 daltons, 250,000 daltons, 300,000 daltons, 350,000 daltons, 400,000 daltons, 450,000 daltons, 500,000 daltons, 550,000 daltons, 600,000 daltons, 650,000 daltons, 700,000 daltons, 750,000 daltons, 800,000 daltons, 850,000 daltons, 900,000 daltons, 950,000 daltons, 1,000,000 daltons, 1,100,000 daltons, 1,200,000 daltons, 1,300,000 daltons, 1,400,000 daltons, 1,500,000 daltons, 1,600,000 daltons, 1,700,000 daltons, 1,800,000 daltons, 1,900,000 daltons, 2,000,000 daltons, or a range between any two of the aforementioned numerical values.

According to an embodiment of the present disclosure, the pharmaceutical composition further comprises water. Preferably, the water is selected from water for injection.

According to an embodiment of the present disclosure, the (i) triamcinolone acetonide active ingredient accounts for 1.0% to 8.0% (w/w) of the total amount of the composition, e.g., 1.0%, 1.5%, 2.0%, 2.5%, 3.0%, 3.5%, 4.0%, 4.5%, 5.0%, 5.5%, 6.0%, 6.5%, 7.0%, 7.5%, 8.0% (w/w), or a range between any two of the aforementioned numerical values. Preferably, the (i) accounts for 3.0% to 5.0% (w/w) of the total amount of the composition.

According to an embodiment of the present disclosure, the (ii) hyaluronic acid or the salt thereof accounts for 0.10% to 5.00% (w/w) of the total amount of the composition, e.g., 0.10%, 0.11%, 0.12%, 0.13%, 0.14%, 0.15%, 0.16%, 0.17%, 0.18%, 0.19%, 0.20%, 0.21%, 0.22%, 0.23%, 0.24%, 0.25%, 0.26%, 0.27%, 0.28%, 0.29%, 0.30%, 0.31%, 0.32%, 0.33%, 0.34%, 0.35%, 0.36%, 0.37%, 0.38%, 0.39%, 0.40%, 0.50%, 0.55%, 0.60%, 0.65%, 0.70%, 0.75%, 0.80%, 0.85%, 0.90%, 0.95%, 1.00%, 1.10%, 1.20%, 1.30%, 1.40%, 1.50%, 1.60%, 1.70%, 1.80%, 1.90%, 2.00%, 2.10%, 2.20%, 2.30%, 2.40%, 2.50%, 2.60%, 2.70%, 2.80%, 2.90%, 3.00%, 3.10%, 3.20%, 3.30%, 3.40%, 3.50%, 3.60%, 3.70%, 3.80%, 3.90%, 4.00%, 4.10%, 4.20%, 4.30%, 4.40%, 4.50%, 4.60%, 4.70%, 4.80%, 4.90%, 5.00% (w/w), or a range between any two of the aforementioned numerical values.

According to an embodiment of the present disclosure, the (iii) buffering agent accounts for 0.05% to 0.80% (w/w) of the total amount of the composition, e.g., 0.05%, 0.10%, 0.15%, 0.20%, 0.25%, 0.30%, 0.35%, 0.40%, 0.45%, 0.50%, 0.55%, 0.60%, 0.65%, 0.70%, 0.75%, 0.80% (w/w), or a range between any two of the aforementioned numerical values. Preferably, the buffering agent accounts for 0.10% to 0.50% (w/w) of the total amount of the composition. In some embodiments, when the buffering agent is selected from the combination of disodium hydrogen phosphate and sodium dihydrogen phosphate, the sodium dihydrogen phosphate accounts for 0.10% to 0.50% (w/w) of the total amount of the composition, e.g., 0.10%, 0.15%, 0.20%, 0.25%, 0.30%, 0.35%, 0.40%, 0.45%, or 0.50%, and the disodium hydrogen phosphate accounts for 0.05% to 0.20% (w/w) of the total amount of the composition, e.g., 0.05%, 0.10%, 0.15%, or 0.20% (w/w).

According to an embodiment of the present disclosure, the (iv) tonicity adjustment accounts for 5.0% to 10.0% (w/w) of the total amount of the composition, e.g., 5.0%, 5.5%, 6.0%, 6.5%, 7.0%, 7.5%, 8.0%, 8.5%, 9.0%, 9.5%, 10.0% (w/w), or a range between any two of the aforementioned numerical values. Preferably, the tonicity adjustment accounts for 6.0% to 9.0% (w/w) of the total amount of the composition.

In certain embodiments, the pharmaceutical composition comprises, by weight percentage, the following ingredients:

| | |
|---|---|
| triamcinolone acetonide active ingredient: | 3.0% to 5.0%; |
| sodium hyaluronate: | 0.1% to 5%; |
| sodium chloride: | 0.6% to 0.8%; |
| sodium dihydrogen phosphate: | 0.2% to 0.4%; |
| disodium hydrogen phosphate: | 0.05% to 0.15%; and |
| sodium hydroxide: q.s., | |
| the balance being water. | |

According to an embodiment of the present disclosure, the pharmaceutical composition has a pH of 6.0 to 8.0, e.g., 6.0, 6.1, 6.2, 6.3, 6.4, 6.5, 6.6, 6.7, 6.8, 6.9, 7.0, 7.1, 7.2, 7.3, 7.4, 7.5, 7.6, 7.7, 7.8, 7.9, 8.0, or a range between any two of the aforementioned numerical values, preferably 6.5 to 8.0. According to an embodiment of the present disclosure, the pharmaceutical composition further comprises a pH regulator selected from sodium hydroxide, sodium bicarbonate, hydrochloric acid, and phosphoric acid. Preferably, the regulator is sodium hydroxide; more preferably, the sodium hydroxide is at a concentration of 1.5 mol/L to 2.5 mol/L, e.g., 2.0 mol/L.

According to an embodiment of the present disclosure, the pharmaceutical composition has an osmotic pressure of 200 mOsm/kg to 400 mOsm/kg, e.g., 200 mOsm/kg, 210 mOsm/kg, 220 mOsm/kg, 230 mOsm/kg, 240 mOsm/kg, 250 mOsm/kg, 260 mOsm/kg, 270 mOsm/kg, 280 mOsm/kg, 290 mOsm/kg, 300 mOsm/kg, 310 mOsm/kg, 320 mOsm/kg, 330 mOsm/kg, 340 mOsm/kg, 350 mOsm/kg, 360 mOsm/kg, 370 mOsm/kg, 380 mOsm/kg, 390 mOsm/kg, 400 mOsm/kg, or a range between any two of the aforementioned numerical values, preferably 250 mOsm/kg to 350 mOsm/kg, and more preferably, 210 mOsm/kg to 325 mOsm/kg.

According to an embodiment of the present disclosure, the pharmaceutical composition is a suspension.

According to an embodiment of the present disclosure, in the pharmaceutical composition, the (i) triamcinolone acetonide active ingredient has an average volume particle size of 0.5 µm to 3.5 µm, e.g., 0.5 µm, 0.6 µm, 0.7 µm, 0.8 µm, 0.9 µm, 1.0 µm, 1.1 µm, 1.2 µm, 1.3 µm, 1.4 µm, 1.5 µm, 1.6 µm, 1.7 µm, 1.8 µm, 1.9 µm, 2.0 µm, 2.1 µm, 2.2 µm, 2.3 µm, 2.4 µm, 2.5 µm, 2.6 µm, 2.7 µm, 2.8 µm, 2.9 µm, 3.0 µm, 3.1 µm, 3.2 µm, 3.3 µm, 3.4 µm, or 3.5 µm; in some embodiments, the (i) triamcinolone acetonide active ingredient has a D₁₀ of 0.4 µm to 1.0 µm, a D₅₀ of 1.0 µm to 2.0 µm, and a D₉₀ of 2.0 µm to 3.8 µm; for example, the D₁₀ is 0.4 µm, 0.5 µm, 0.6 µm, 0.7 µm, 0.8 µm, 0.9 µm, or 1.0 µm; the D₅₀ is 1.0 µm, 1.1 µm, 1.2 µm, 1.3 µm, 1.4 µm, 1.5 µm, 1.6 µm, 1.7 µm, 1.8 µm, 1.9 µm, or 2.0 µm; the D₉₀ is 2.0 µm, 2.1 µm, 2.2 µm, 2.3 µm, 2.4 µm, 2.5 µm, 2.6 µm, 2.7 µm, 2.8 µm, 2.9 µm, 3.0 µm, 3.1 µm, 3.2 µm, 3.3 µm, 3.4 µm, 3.5 µm, 3.6 µm, 3.7 µm, or 3.8 µm. According to certain embodiments of the present disclosure, the pharmaceutical composition is a suspension comprising a particle group of the (i) triamcinolone acetonide active ingredient, wherein the particle group has a D₁₀ of 0.4 µm to 1.0 µm, a D₅₀ of 1.0 µm to 2.0 µm, and a D₉₀ of 2.0 µm to 3.8 µm. In some embodiments, the particle group in the pharmaceutical composition has a D₁₀ of 0.6 µm to 0.85 µm, a D₅₀ of 1.5 µm to 1.8 µm, and a D₉₀ of 3.0 µm to 3.5 µm. In some embodiments, the particle group in the pharmaceutical composition has a D₁₀ of 0.7 µm to 0.8 µm, a D₅₀ of 1.6 µm to 1.8 µm, and a D₉₀ of 3.1 µm to 3.5 µm.

According to certain embodiments of the present disclosure, in the pharmaceutical composition, 10% of the triamcinolone acetonide active ingredient particles have a particle size less than 0.4 µm to 1.0 µm, 50% of the triamcinolone acetonide active ingredient particles have a particle size less than 1.0 µm to 2.0 µm, and 90% of the triamcinolone acetonide active ingredient particles have a particle size less than 2.0 µm to 3.8 µm. As an example, in the pharmaceutical composition, when the D₁₀ is 0.4 µm, the D₅₀ is 1.0 µm, and the D₉₀ is 2.0 µm, 10% of the triamcinolone acetonide active ingredient particles have a particle size less than 0.4 µm, 50% of the triamcinolone acetonide active ingredient particles have a particle size less than 1.0 µm, and 90% of the triamcinolone acetonide active ingredient particles have a particle size less than 2.0 µm. As another example, in the pharmaceutical composition, when the D₁₀ is 1.0 µm, the D₅₀ is 2.0 µm, and the D₉₀ is 3.8 µm, 10% of the triamcinolone acetonide active ingredient particles have a particle size less than 1.0 µm, 50% of the triamcinolone acetonide active ingredient particles have a particle size less than 2.0 µm, and 90% of the triamcinolone acetonide active ingredient particles have a particle size less than 3.8 µm. According to certain embodiments of the present disclosure, in the pharmaceutical composition, 10% of the triamcinolone acetonide active ingredient particles have a particle size less than 0.6 µm to 0.85 µm, 50% of the triamcinolone acetonide active ingredient particles have a particle size less than 1.5 µm to 1.8 µm, 90% of the triamcinolone acetonide active ingredient particles have a particle size less than 3.0 µm to 3.5 µm, and the particle group has an average volume particle size of about 1.5 µm to about 2.5 µm. According to certain embodiments of the present disclosure, in the pharmaceutical composition, 10% of the triamcinolone acetonide active ingredient particles have a particle size less than 0.7 µm to 0.8 µm, 50% of the triamcinolone acetonide active ingredient particles have a particle size less than 1.6 µm to 1.8 µm, 90% of the triamcinolone acetonide active ingredient particles have a particle size less than 3.1 µm to 3.5 µm, and the particle group has an average volume particle size of about 1.8 µm to about 2.0 µm.

According to certain embodiments of the present disclosure, in the pharmaceutical composition, 10% of the triamcinolone acetonide active ingredient particles have a particle size less than about 0.5 µm to about 0.85 µm, 50% of the triamcinolone acetonide active ingredient particles have a particle size less than about 1.2 µm to about 1.9 µm, and 90% of the triamcinolone acetonide particles have a particle size less than about 2.5 µm to about 3.6 µm. According to certain embodiments of the present disclosure, in the pharmaceutical composition, 10% of the triamcinolone acetonide active ingredient particles have a particle size less than about 0.7 µm to about 0.8 µm, 50% of the triamcinolone acetonide particles have a particle size less than about 1.5 µm to about 1.8 µm, and 90% of the triamcinolone acetonide active ingredient particles have a particle size less than about 3.0 µm to about 3.5 µm. According to certain embodiments of the present disclosure, in the pharmaceutical composition, 10% of the triamcinolone acetonide active ingredient particles have a particle size less than about 0.75 µm to about 0.78 µm, 50% of the triamcinolone acetonide particles have a particle size less than about 1.63 µm to about 1.79 µm, and 90% of the triamcinolone acetonide active ingredient particles have a particle size less than about 3.15 µm to about 3.48 µm. According to certain embodiments of the present disclosure, in the pharmaceutical composition, the triamcinolone acetonide active ingredient particle group has an average volume particle size of about 0.5 µm to about 2.5 µm. According to certain embodiments of the present disclosure, in the pharmaceutical composition, the triamcinolone acetonide active ingredient particle group has an average volume particle size of about 0.7 µm to about 2.1 µm. According to certain embodiments of the present disclosure, in the pharmaceutical composition, the triamcinolone acetonide active ingredient particle group has an average volume particle size of about 0.8 µm to about 2.0 µm. According to certain embodiments of the present disclosure, in the pharmaceutical composition, the triamcinolone acetonide active ingredient particle group has an average volume particle size of about 0.81 µm to about 1.99 µm. According to certain embodiments of the present disclosure, the pharmaceutical composition is subjected to a wet milling step. For example, the pharmaceutical composition may be subjected to a ball milling step. As an example, hyaluronic acid, e.g., in the form of an aqueous hyaluronic acid solution, may be added to a composition comprising triamcinolone acetonide particles. As another example, triamcinolone acetonide may be added to a composition comprising hyaluronic acid, e.g., an aqueous solution comprising hyaluronic acid. Subsequently, the triamcinolone acetonide active ingredient particles may be ground in the composition comprising hyaluronic acid until the effective average particle size falls in the desired range, e.g., 0.5 µm, 0.6 µm, 0.7 µm, 0.8 µm, 0.9 µm, 1.0 µm, 1.1 µm, 1.2 µm, 1.3 µm, 1.4 µm, 1.5 µm, 1.6 µm, 1.7 µm, 1.8 µm, 1.9 µm, 2.0 µm, 2.1 µm, 2.2 µm, 2.3 µm, 2.4 µm, 2.5 µm, 2.6 µm, 2.7 µm, 2.8 µm, 2.9 µm, 3.0 µm, 3.1 µm, 3.2 µm, 3.3 µm, 3.4 µm, 3.5 µm, or a range between any two of the aforementioned numerical values.

According to certain embodiments of the present disclosure, the pharmaceutical composition comprises substantially no triamcinolone acetonide active ingredient particle group having an effective average particle size less than 0.2 µm. According to certain embodiments of the present disclosure, the pharmaceutical composition comprises substantially no triamcinolone acetonide active ingredient particle group having an effective average particle size of 0.2 µm to 0.4 µm. According to certain embodiments of the present disclosure, in the pharmaceutical composition, more than 10%, more than 50%, or more than 90% of the triamcinolone acetonide active ingredient particles have a particle size greater than 0.2 µm, greater than 0.3 µm, greater than 0.4 µm, greater than 0.5 µm, greater than 0.6 µm, greater than 0.7 µm, greater than 0.8 µm, greater than 0.9 µm, greater than 1.0 µm, greater than 1.5 µm, greater than 2.0 µm, greater than 2.5 µm, greater than 3.0 µm, or greater than 3.8 µm. According to certain embodiments of the present disclosure, in the pharmaceutical composition, about or more than 10% of the triamcinolone acetonide active ingredient particles have a particle size greater than 3.8 µm. According to certain embodiments of the present disclosure, in the pharmaceutical composition, about or more than 50% of the triamcinolone acetonide active ingredient particles have a particle size greater than 2.0 µm. According to certain embodiments of the present disclosure, in the pharmaceutical composition, about or more than 90% of the triamcinolone acetonide active ingredient particles have a particle size greater than 1.0 µm. According to certain embodiments of the present disclosure, in the pharmaceutical composition, about or more than 10% of the triamcinolone acetonide active ingredient particles have a particle size greater than 2.0 µm, about or more than 50% of the triamcinolone acetonide active ingredient particles have a particle size greater than 1.0 µm, and about or more than 90% of the triamcinolone acetonide active ingredient particles have a particle size greater than 0.4 µm.

According to an embodiment of the present disclosure, the pharmaceutical composition may be an injectable liquid and administered topically; preferably, the pharmaceutical composition is administered through the suprachoroidal space.

In another aspect, the present disclosure provides a preparation method for the composition, comprising the following steps:
(a) mixing the hyaluronic acid or the salt thereof, the buffering agent, and the tonicity adjustment in water and optionally adding a pH regulator; and
(b) adding the triamcinolone acetonide active ingredient into the solution obtained in the step (a) described above and dispersing the triamcinolone acetonide active ingredient.

According to an embodiment of the present disclosure, in step (b), a drug substance is dispersed homogeneously under stirring.

According to an embodiment of the present disclosure, the preparation method further comprises step (c): wet-milling the suspension obtained in step (b). In some embodiments, the wet milling process is performed using a wet mill, with the equipment temperature controlled at 4 °C to 8 °C (e.g., 6 °C), the pump speed being 100 rpm to 120 rpm (e.g., 110 rpm), the main unit rotation speed being 2000 rpm to 3000 rpm (e.g., 2500 rpm), and the wet milling cycle count being 4 to 8 (e.g., 6).

### Beneficial Effects

The present disclosure provides a triamcinolone acetonide composition that features a simple formulation process and good auxiliary material compatibility, has suitable viscosity, osmotic pressure, and particle size ranges, is easily injected, particularly administered by suprachoroidal space injection, and has good stability, safety, and bioavailability.

### DETAILED DESCRIPTION

The technical solutions of the present disclosure are further described in detail below with reference to specific examples. It will be appreciated that the following examples are merely exemplary illustrations and explanations of the present disclosure and should not be construed as limiting the claimed scope of the present disclosure. All techniques implemented on the basis of the above content of the present disclosure fall within the claimed scope of the present disclosure.

Unless otherwise stated, the starting materials and reagents used in the following examples are all commercially available products or can be prepared by using known methods.

### Example 1. Preparation of Triamcinolone Acetonide Composition

| Formula F-1 | Amount | |
|---|---|---|
| | Amount per unit formula/mg | wt% |
| Triamcinolone acetonide | 40 | 4.0 |
| Sodium hyaluronate | 2.3 | 0.23 |
| Sodium chloride | 7.96 | 0.796 |
| Sodium dihydrogen phosphate (monohydrate) | 3.45 | 0.345 |
| Disodium hydrogen phosphate (dodecahydrate) | 1.0 | 0.1 |
| Sodium hydroxide | q.s. | / |
| Water for injection | 985.29 | / |

The manufacturing process of F-1: The formula amounts of auxiliary materials were weighed out, placed in the formula amount of water, and dissolved using an electric mixer, and the pH of the solution was adjusted to 6.5 to 7.5 using a 2 mol/L sodium hydride solution. The formula amount of triamcinolone acetonide drug substance was slowly added under stirring and homogeneously dispersed by stirring. The resulting suspension in which triamcinolone acetonide was homogeneously dispersed was subjected to wet milling cycles. When a stable and continuous white liquid flowed out, the liquid was collected, and the particle size of the sample was monitored and controlled. The wet milling process was performed using a wet mill at a temperature of 6 °C, with the pump speed being 110 rpm and the main unit rotation speed being 2500 rpm.

### Example 2. Triamcinolone Acetonide Injection

A triamcinolone acetonide suspension was prepared according to Example 1, bottled (1 mL/vial), and sterilized.

### Example 3. Triamcinolone Acetonide Injection Stability Tests

| Formula No. | | Limits | F-1 | F-1 | F-1 |
|---|---|---|---|---|---|
| Test conditions | | / | Roomtemperature sample | 40 °C/RH75% - 1 month | 40 °C/RH75% - 3 months |
| Appearance | | The sample was a suspension of fine particles. After the sample was left to stand, the fine particles settled. After the sample was shaken, a homogeneous milky white suspension was formed. | The sample was a suspension of fine particles. After the sample was left to stand, the fine particles settled. After the sample was shaken, a homogeneous milky white suspension was formed. | The sample was a suspension of fine particles. After the sample was left to stand, the fine particles settled. After the sample was shaken, a homogeneous milky white suspension was formed. | The sample was a suspension of fine particles. After the sample was left to stand, the fine particles settled. After the sample was shaken, a homogeneous milky white suspension was formed. |
| pH value | | 6.5~7.5 | 7.09 (18.8 °C) | 7.10 (18.8 °C) | 7.18 (24.7 °C) |
| Osmotic pressure (mOsm/kg) | | 280~320 | 320 | 318 | 318.5 |
| Particle size VMD (µm) | | 1~3 | D10=0.78 µm | D10=0.78 µm | D10=0.75 µm |
| | | | D50=1.76 µm | D50=1.79 µm | D50=1.63 µm |
| | | | D90=3.41 µm | D90=3.48 µm | D90=3.15 µm |
| | | | VMD=1.93 µm | VMD=1.99 µm | VMD=1.81 µm |
| Triamcinolone acetonide content (%, w/w) | | 3.5~4.2 | 3.8 | 3.8 | 3.9 |
| Related substances (ChP2020) | Impurity A | ≤0.5% | 0.02 | 0.02 | 0.02 |
| | Impurity C | ≤0.5% | 0.18 | 0.08 | 0.21 |
| | Impurity B | ≤0.5% | 0.04 | 0.04 | 0.04 |
| | Maximum unknown single impurity | ≤0.5% | 0.09 | 0.01 | 0.06 |
| | Total impurity | ≤1.5% | 0.38 | 0.21 | 0.40 |

The embodiments of the technical solutions of the present disclosure have been described above by way of example. It will be appreciated that the claimed scope of the present disclosure is not limited to the embodiments described above. Any modification, equivalent replacement, improvement, and the like made by those skilled in the art without departing from the spirit and principle of the present disclosure shall fall within the claimed scope of the present application.

## Claims

1. A pharmaceutical composition, comprising the following ingredients:
(i) a triamcinolone acetonide active ingredient;
(ii) hyaluronic acid or a salt thereof;
(iii) a buffering agent; and
(iv) a tonicity adjustment.

2. The pharmaceutical composition as claimed in claim 1, wherein the salt of hyaluronic acid may be selected from a hyaluronic acid metal salt, preferably from one or more of a hyaluronic acid alkali metal salt and a hyaluronic acid alkaline earth metal salt; more preferably, the salt of hyaluronic acid is selected from sodium hyaluronate; particularly preferably, the hyaluronic acid or the salt thereof has a molecular weight of 50,000 daltons to 2,000,000 daltons.

3. The pharmaceutical composition as claimed in claim 1 or 2, wherein the buffering agent is selected from one or more of an acetate, a citrate, a phosphate, and a borate; preferably, the buffering agent is selected from a phosphate buffering agent; more preferably, the buffering agent is selected from one or a combination of two of disodium hydrogen phosphate and sodium dihydrogen phosphate; the sodium dihydrogen phosphate may be selected from sodium dihydrogen phosphate monohydrate, and the disodium hydrogen phosphate may be selected from disodium hydrogen phosphate dodecahydrate.

4. The pharmaceutical composition as claimed in any one of claims 1 to 3, wherein the tonicity adjustment is selected from one or a combination of two of sodium chloride and potassium chloride.

5. The pharmaceutical composition as claimed in any one of claims 1 to 4, further comprising water, wherein preferably, the water is selected from water for injection.

6. The pharmaceutical composition as claimed in any one of claims 1 to 5, wherein the (i) triamcinolone acetonide active ingredient accounts for 1.0% to 8.0% (w/w) of the total amount of the composition; the (ii) hyaluronic acid or the salt thereof accounts for 0.10% to 5.00% (w/w) of the total amount of the composition; the (iii) buffering agent accounts for 0.05% to 0.80% (w/w) of the total amount of the composition; the (iv) tonicity adjustment accounts for 5.0% to 10.0% (w/w) of the total amount of the composition.

7. The pharmaceutical composition as claimed in any one of claims 1 to 6, comprising, by weight percentage, the following ingredients:
triamcinolone acetonide active ingredient: 3.0% to 5.0%;
sodium hyaluronate: 0.1% to 5%;
sodium chloride: 0.6% to 0.8%;
sodium dihydrogen phosphate: 0.2% to 0.4%;
disodium hydrogen phosphate: 0.05% to 0.15%; and
sodium hydroxide: q.s.,
the balance being water.

8. The pharmaceutical composition as claimed in any one of claims 1 to 7, having a pH of 6.0 to 8.0.

9. The pharmaceutical composition as claimed in any one of claims 1 to 8, having an osmotic pressure of 200 mOsm/kg to 400 mOsm/kg, wherein in the pharmaceutical composition, the triamcinolone acetonide active ingredient has an average volume particle size of 0.5 µm to 3.5 µm.

10. A preparation method for the pharmaceutical composition as claimed in any one of claims 1 to 9, comprising the following steps:
(a) mixing the hyaluronic acid or the salt thereof, the buffering agent, and the tonicity adjustment in water and optionally adding a pH regulator; and
(b) adding the triamcinolone acetonide active ingredient into the solution obtained in the step (a) described above and dispersing the triamcinolone acetonide active ingredient,
wherein preferably, in step (b), a drug substance is dispersed homogeneously under stirring; preferably, the preparation method further comprises step (c): wet-milling the suspension obtained in step (b).
